# EUROPEAN PATENT SPECIFICATION

(11) **EP 2 182 358 B1**
(45) Date of publication and mention of the grant of the patent: **04.07.2012**
(21) Application number: 09174415.1
(22) Date of filing: 29.10.2009
(51) Int. Cl.: G01N 27/30, G01N 27/48, G01N 33/543

(54) **Method for detecting analyte, detection apparatus, and test chip**
Verfahren zur Analytdetektion, Detektionsvorrichtung und Prüfchip
Procédé pour détecter une analyse, appareil de détection et puce de test

(30) Priority: 30.10.2008 JP 2008279385
(43) Date of publication of application: 05.05.2010
(73) Proprietor: SYSMEX CORPORATION, Kobe-shi, Hyogo 651-0073 (JP)
(72) Inventor: Iwanaga, Shigeki, Kobe-shi Hyogo 651-0073 (JP); Kirimura, Hiroya, Kobe-shi Hyogo 651-0073 (JP)
(74) Representative: Paemen, Liesbet R.J.

(56) References cited:
- EP-A1- 1 947 452
- WO-A1-95/19566
- WO-A1-99/54718
- WO-A2-2004/046369
- US-A- 5 766 934
- US-A1- 2005 142 033
- US-A1- 2008 081 329
- PAPAGEORGIOU N ET AL: "Iodine/triiodide reduction electrocatalyst for aqueous and organic media" March 1997 (1997-03), JOURNAL OF THE ELECTROCHEMICAL SOCIETY 1997 MAR ELECTROCHEMICAL SOC INC, VOL. 144, NR. 3, PAGE(S) 876 - 884 , XP002566507 * abstract * * page 877, left-hand column, paragraph 2 - page 878, left-hand column, paragraph 3 * * figure 3 *
- AN H J ET AL: "Cationic surfactant promoted reductive electrodeposition of nanocrystalline anatase TiO2 for application to dye-sensitized solar cells" ELECTROCHIMICA ACTA, ELSEVIER SCIENCE PUBLISHERS, BARKING, GB, vol. 50, no. 13, 30 April 2005 (2005-04-30), pages 2713-2718, XP004843814 ISSN: 0013-4686
- BETTELHEIM A ET AL: "Application of a polymer solid electrolyte for the vapor-phase electrocatalysis of dioxygen reduction by some cobalt porphyrins" JOURNAL OF ELECTROANALYTICAL CHEMISTRY AND INTERFACIALELECTRO CHEMISTRY, ELSEVIER, AMSTERDAM, NL, vol. 238, no. 1-2, 10 December 1987 (1987-12-10), pages 259-276, XP026518116 ISSN: 0022-0728 [retrieved on 1987-12-10]

## Description

### FIELD OF THE INVENTION

The present invention relates to a method for detecting an analyte, a detection apparatus, and a test chip.

### BACKGROUND

In clinical examination and diagnosis of diseases, disease-derived genes, proteins and the like contained in biological samples are detected by gene detection methods and immunological detection methods. Specific examples include immunochromatography, latex agglutination, enzyme immunoassay, chemiluminescent immunoassay, gene amplification PCR, and the like. In these detection methods, however, there is room for improvement from the viewpoint of simplicity, rapidity, and cost.

Consequently, International Publication No. 2007/037341 has proposed a method wherein a current generated from a sensitizing dye by photoexcitation is utilized in detecting an analyte. In this method, a semiconductor layer (electron-receiving layer) is formed on an electrode, and a probe substance capable of binding to the analyte is immobilized on the semiconductor layer. Then, the analyte modified with a sensitizing dye is trapped with the probe substance, and then the sensitizing dye with which the analyte has been modified is irradiated with a light for exciting the sensitizing dye. As a result, electrons are emitted from the sensitizing dye with which the analyte has been modified, and a current generated by the released electrons upon being received by the semiconductor layer is detected. In the conventional method, however, the detection efficiency of a current is low, so there has been demand for improvements in the detection sensitivity of an analyte.

Papageorgiou and coworkers (J Electrochem Soc 1997; 144(3):876-884) report on photoelectrochemical solar energy conversion systems and electrochemical sensor devices utilizing iodide/triiodide redox couples as mediators. Performance limitations are minimized by using catalytic nanosized platinum metal clusters coated on the counter electrode.

### SUMMARY OF THE INVENTION

The scope of the present invention is defined solely by the appended claims, and is not affected to any degree by the statements within this summary.
(1) A first aspect of the present invention is a method for detecting an analyte modified with a modulator releasing electrons upon photoexcitation, comprising:
   trapping an analyte in a sample by using a test chip, wherein the test chip comprises: a working electrode including a semiconductor layer, a probe being capable of trapping the analyte and being immobilized on the semiconductor layer, a counter electrode consisting of an electroconductive material, and a reduction electrode for reducing an electrolyte contained in an electrolyte medium to be contacted with the working electrode and the counter electrode, wherein the reduction electrode is arranged separately from the counter electrode ; irradiating, with a light for exciting the modulator, the modulator with which the analyte trapped by the probe has been modified, while the working electrode, the counter electrode and the reduction electrode are contacted with an electrolyte medium containing the electrolyte; measuring a current which due to movement of electrons from the photoexcited modulator to the working electrode passes between the working electrode and the counter electrode, while the electrolyte contained in the electrolyte medium is reduced by the reduction electrode. According to the detection method of the present invention, the reduction reaction of an electrolyte can be promoted by reducing the electrolyte with a reduction electrode arranged in a test chip, thereby increasing a current passing between a working electrode and a counter electrode, to enable increase in the detection sensitivity of an analyte.
(2) According to particular embodiments, reducing the electrolyte by the reduction electrode in the methods (1) of the invention is carried out with the reduction electrode under application of a potential. According to the detection methods of the present invention, the reduction reaction of an electrolyte can be promoted by applying a potential to a reduction electrode, thereby increasing a current passing between a working electrode and a counter electrode, to enable increase in the detection sensitivity of an analyte.
(3) According to further particular embodiments, reducing the electrolyte by the reduction electrode in the methods (2) of the invention is carried out with the reduction electrode under application of a potential of -0.5 V or more and less than 0 V relative to the counter electrode.
(4) According to particular embodiments, the reduction electrode in the methods (1) of the invention is kept at natural potential capable of reducing the electrolyte. According to the detection methods of the present invention, even if a potential is not positively applied by a power supply or the like, the reduction of an electrolyte can be promoted by only the presence of a reduction electrode, thereby increasing a current passing between a working electrode and a counter electrode, to enable increase in the detection sensitivity of an analyte.
(5) According to certain specific embodiments of the methods (1) to (4) of the invention the probe is a nucleic acid, a protein or a peptide.
(6) According to further specific embodiments of the methods (1) to (5) the electrolyte is iodine or an iodide.
(7) A second aspect of the present invention is an apparatus for detecting an analyte modified with a modulator releasing electrons upon photoexcitation, comprising:
   a test chip receiving part capable of receiving a test chip; a light source for irradiating, with a light for exciting the modulator, the modulator with which the analyte has been modified in the test chip received by the test chip receiving part; a power supply for supplying a potential to the reduction electrode; and an electric current measuring part for measuring a current which due to movement of electrons from the modulator excited by the light emitted from the light source to the working electrode, passes between the working electrode and the counter electrode, wherein the testchip comprises: a working electrode including a semiconductor layer, a probe being capable of trapping the analyte and being immobilized on the semiconductor layer, a counter electrode consisting of an electroconductive material, and a reduction electrode for reducing an electrolyte contained in an electrolyte medium to be contacted with the working electrode and the counter electrode, wherein the reduction electrode is arranged separately from the counter electrode .

   By this constitution, the reduction reaction of an electrolyte can be promoted by applying a potential to the reduction electrode arranged in the test chip, thereby increasing a current passing between the working electrode and the counter electrode, to enable increase in the detection sensitivity of an analyte.
(8) According to particular embodiments, the power supply for supplying a potential in the apparatus (7) of the invention, applies to the reduction electrode, a potential of -0.5 V or more and less than 0 V relative to the counter electrode.
(9) According to further particular embodiments, the light source in the apparatus (7) or (8) of the invention generates a light of wavelength exciting the modulator for modifying an analyte.
(10) A third aspect of the present invention is a test chip for detecting an analyte modified with a modulator releasing electrons upon photoexcitation, comprising; a working electrode including a semiconductor layer; a probe being capable of trapping the analyte and being immobilized on the semiconductor layer; a counter electrode consisting of an electroconductive material; and a reduction electrode for reducing an electrolyte contained in the electrolyte medium to be contacted with the working electrode and the counter electrode, wherein the reduction electrode is arranged separately from the counter electrode .
   By this constitution, the reduction reaction of an electrolyte can be promoted by the reduction electrode arranged in the test chip, thereby increasing a current passing between the working electrode and the counter electrode, to enable increase in the detection sensitivity of an analyte.
(11) According to particular embodiments, the reduction electrode in the test chip (10) of the invention is an electrode consisting of a comb-like structure with several parallel long lines.
(12) According to particular embodiments, the electrolyte in the test chips (10) or (11) of the invention is iodine or an iodide.
(13) According to particular embodiments, the probe in the test chips (10) to (12) of the invention is a nucleic acid, a protein or a peptide.
(14) In specific embodiments, the probe in the test chips (10) to (13) of the invention is immobilized by chemical adsorption onto the semiconductor layer.
(15) In further specific embodiments, the probe in the test chip (14) of the invention has a thiol group as a binding group for chemical adsorption to the semiconductor layer.

### BRIEF DESCRIPTION OF THE DRAWINGS

FIG. 1 is a perspective view showing the detection apparatus in accordance with one embodiment of the present invention;
FIG. 2 is a block diagram showing the constitution of the detection apparatus shown in FIG. 1;
FIG. 3 is a perspective view showing the test chip in accordance with one embodiment of the present invention;
FIG. 4 is a sectional view showing the test chip shown in FIG. 3;
FIG. 5 is a plan view of the test chip in FIG. 3 when viewed from the lower surface side;
FIG. 6 is a plan view of the test chip in FIG. 3 when viewed from the upper surface side;
FIG. 7 is a schematic view schematically showing the test chip shown in FIG. 3;
FIG. 8 is a flowchart showing the procedure of injecting an analyte sample into the test chip by the user;
FIG. 9 is a flowchart showing the procedure of detection operation of the detection apparatus;
FIG. 10 is a graph showing a measurement result of a photocurrent passing between a working electrode and a counter electrode in Experiment 1;
FIG. 11 is a schematic diagram schematically showing the working electrode used in Experiment 2;
FIG. 12 is a graph showing a change with time in the photocurrent value detected in the working electrode in Experiment 2;
FIG. 13 is a graph showing photocurrent values detected in the working electrode, and currents detected in the reduction electrode, in Experiment 2;
FIG. 14 is a graph showing a measurement result in a cyclic voltammogram of an electrolyte in Experiment 3;
FIG. 15 is a graph showing a measurement result of the photocurrent depending on a change in the potential applied to the reduction electrode in Experiment 4; and
FIG. 16 is a graph showing a measurement result of the photocurrent when the distance between the working electrode and the counter electrode is changed in Experiment 5.

### DETAILED DESCRIPTION OF PARTICULAR EMBODIMENTS OF THE INVENTION

Hereinafter, the method for detecting an analyte, the detection apparatus and the test chip of the present invention will be described with reference to the drawings.

### (Constitution of the detection apparatus)

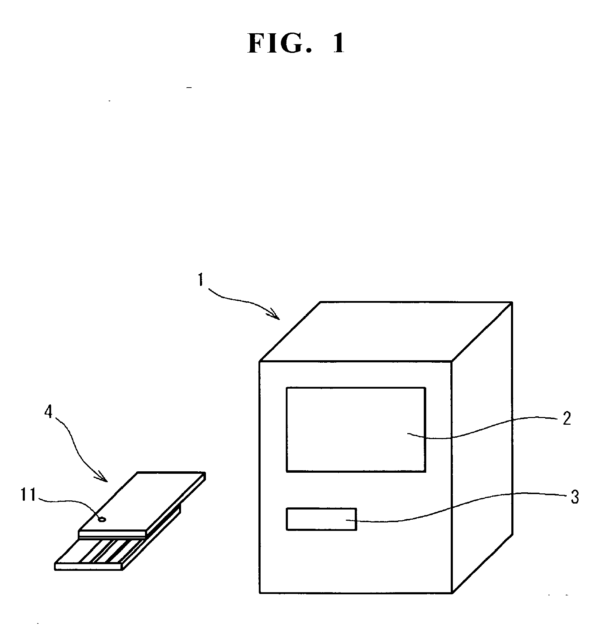

FIG. 1 is a perspective view of the detection apparatus in accordance with one embodiment of the present invention. The detection apparatus 1 is an apparatus for detecting an analyte having specific binding property, such as a nucleic acid, a protein or a peptide collected from living cells or synthesized artificially. The detection apparatus 1 is for example an HPV detection apparatus for detecting, in an analyte sample, mRNA of human papillomavirus (referred to hereinafter as HPV) that is a causative virus for cervical cancer.

The detection apparatus 1 in the embodiment of the present invention includes a chip-receiving part 3 into which a test chip 4 is inserted and a display 2 on which a detection result is displayed. The test chip 4 includes a sample injection port 11 through which an analyte sample is injected into the chip, and has a function of trapping a dye-modified analyte in the analyte sample. The test chip 4 is disposable.

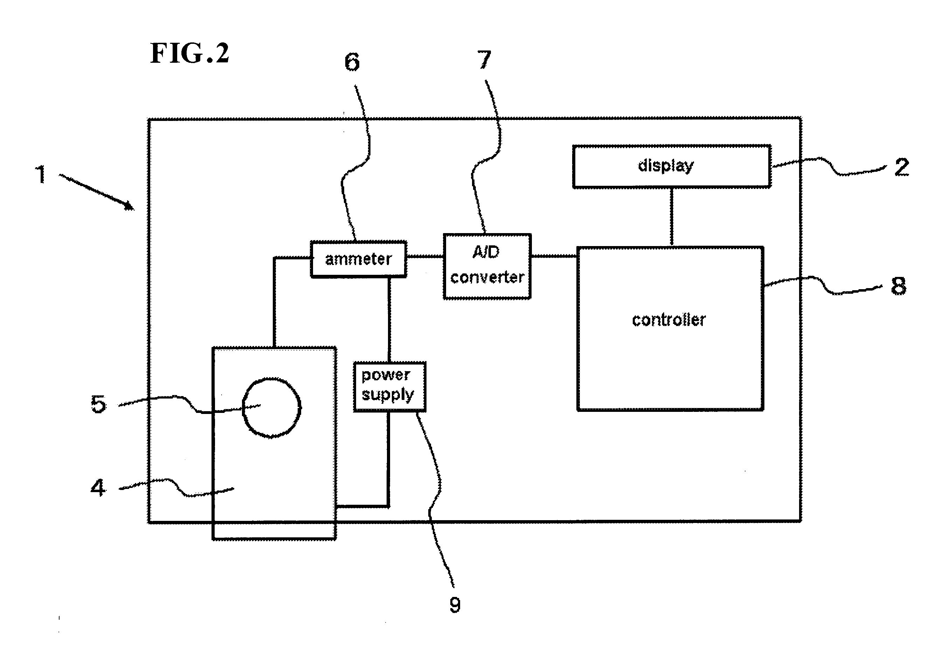

FIG. 2 is a block diagram showing the constitution of the detection apparatus 1. The detection apparatus 1 includes a light source 5, an ammeter (current measuring meter) 6, a power supply (voltage applicator) 9, an A/D converter 7, a controller 8 and a display 2.

The light source 5 applies a light to a dye that has modified an analyte trapped by the test chip 4, thereby exciting the dye. The ammeter 6 measures a current which due to electrons emitted from the excited dye, passes through the test chip 4. The power supply 9 applies a predetermined potential to an electrode arranged in the test chip 4. The A/D converter 7 converts a current value measured by the ammeter 6 into a digital value. The controller 8 is composed of CPU, ROM and RAM, and regulates the operation of the light source 5, the ammeter 6, the power supply 9, and the display 2. On the basis of a previously prepared calibration curve showing the relationship between current values and the amounts of the analyte, the controller 8 makes a rough estimate of the amount of the analyte in an analyte sample, from the digital value into which the current value has been converted by the A/D converter 7. The display 2 displays that amount of the analyte in an analyte sample estimated roughly by the controller 8.

Then, the constitution of the analyte detection test chip 4 in accordance with one embodiment of the present invention, and the method for specifically detecting an analyte by the test chip 4 will be described.

### (Constitution of the test chip)

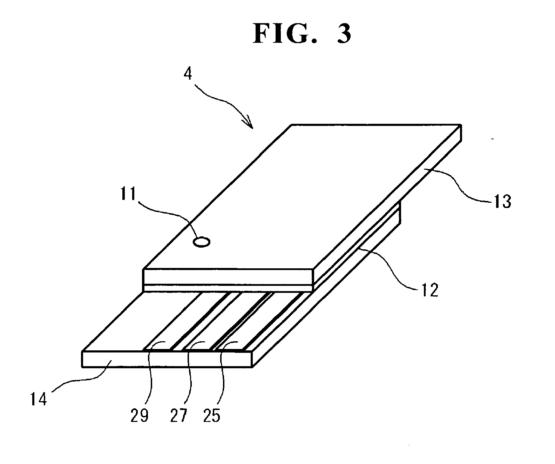

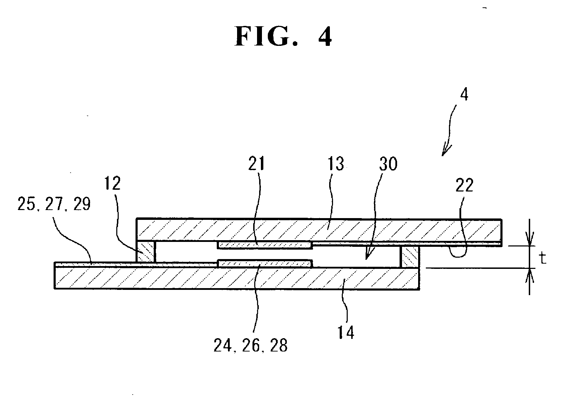

FIG. 3 is a perspective view of the test chip 4 in accordance with certain embodiments of the present invention, and FIG. 4 is a sectional view of the test chip 4. The test chip 4 includes an upper substrate 13, a lower substrate 14 arranged below the upper substrate 13, and a spacing member 12 placed between the upper substrate 13 and the lower substrate 14.

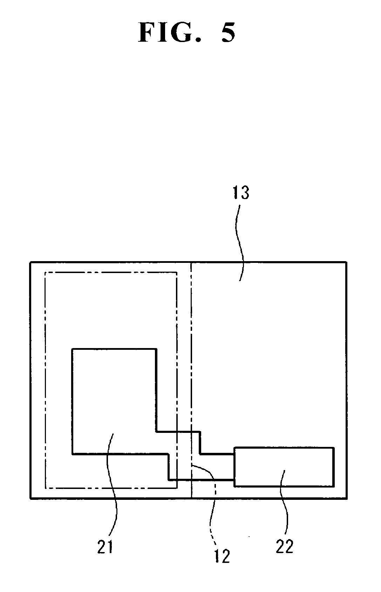

FIG. 5 is a plan view of the upper substrate 13 when viewed from the lower surface side. The upper substrate 13 is formed in a rectangular form, and a working electrode 21 and an electrode lead 22 connected to the working electrode 21 are formed on the surface (lower surface) of the upper substrate 13. The working electrode 21 is formed in an almost rectangular form and arranged on one side (the left side of FIG. 5) of the upper substrate 14, and the electrode lead 22 extends from the working electrode 21 to the other side (the right side of FIG. 5) of the upper substrate 14.

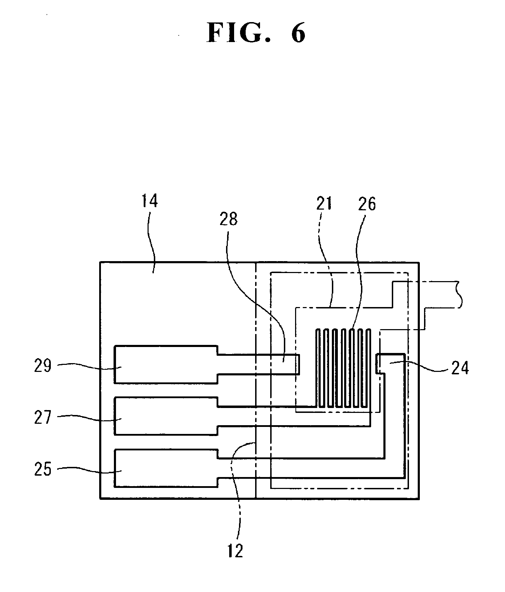

FIG. 6 is a plan view of the lower substrate 14 when viewed from the upper surface side. The lower substrate 14 is formed in a rectangular form with approximately the same dimensions as those of the upper substrate 13, and the lower substrate 14 is provided thereon (upper surface) with a counter electrode 24, an electrode lead 25 connected to this counter electrode 24, a reduction electrode 26, an electrode lead 27 connected to the reduction electrode 26, a reference electrode 28, and an electrode lead 29 connected to the reference electrode 28.

The reduction electrode 26 is formed as a "comb-shaped" electrode having long lines arranged in parallel with one another, has an outline of an almost rectangular form and is arranged on one side (the right side of FIG. 6) of the lower substrate 14. The electrode lead 27 of the reduction electrode 26 extends from the reduction electrode 26 to the other side (the left side of FIG. 6) of the lower substrate.

The counter electrode 24 and the reference electrode 28 are arranged on both sides of the reduction electrode 26 therebetween. The electrode lead 25 of the counter electrode 24, and the electrode lead 29 of the reference electrode 28, each extend from one side of the lower substrate 14 to the other side. The electrode leads 27, 25 and 29 of the reduction electrode 26, the counter electrode 24, and the reference electrode 28 are arranged respectively in parallel with one another at the other side of the lower substrate 14.

As shown in FIGS. 3 and 4, the upper substrate 13 and the lower substrate 14 are arranged to overlap at one side such that the electrodes formed respectively thereon are faced with each another in the vertical direction. The spacing member 12 interposes in the portion where the upper substrate 13 and the lower substrate 14 overlap (are faced) with each other. The electrode leads 22, 25, 27 and 29 formed on the upper substrate 13 and lower substrate 14 are protruded from the overlapping portion of the upper substrate 13 and lower substrate 14 and thereby exposed to the outside.

As shown in FIGS. 5 and 6, the spacing member 12 is formed in the form of a rectangular looped body and composed of an insulating silicone rubber. The spacing member 12 is arranged so as to surround the region where the working electrode 21, the reduction electrode 26, the counter electrode 24 and the reference electrode 28 are faced with each other. A spacing corresponding to the thickness t of the silicone rubber 12 is formed between the upper substrate 13 and the lower substrate 14, and a space 30 (see FIG. 4) for accommodating an analyte sample and an electrolyte is thereby formed among the respective electrodes 21, 24, 26 and 28. The sample injection port 11 formed in the upper substrate 13 is formed by penetrating the upper substrate 13 such that an analyte sample or an electrolyte can be injected to the space 30.

In the embodiment of the present invention, the working electrode 21 is formed on the surface of the upper substrate 13, while the counter electrode 24, the reduction electrode 26 and the reference electrode 28 are formed on the surface of the lower substrate 14, but the positional relationship among the working electrode 21, the counter electrode 24, the reduction electrode 26 and the reference electrode 28 is not particularly limited as long as the respective electrodes are not contacted with another electrode and are arranged in the frame of the spacing member 12. For example, the working electrode 21, the counter electrode 24, the reduction electrode 26 and the reference electrode 28 may be arranged on the same substrate.

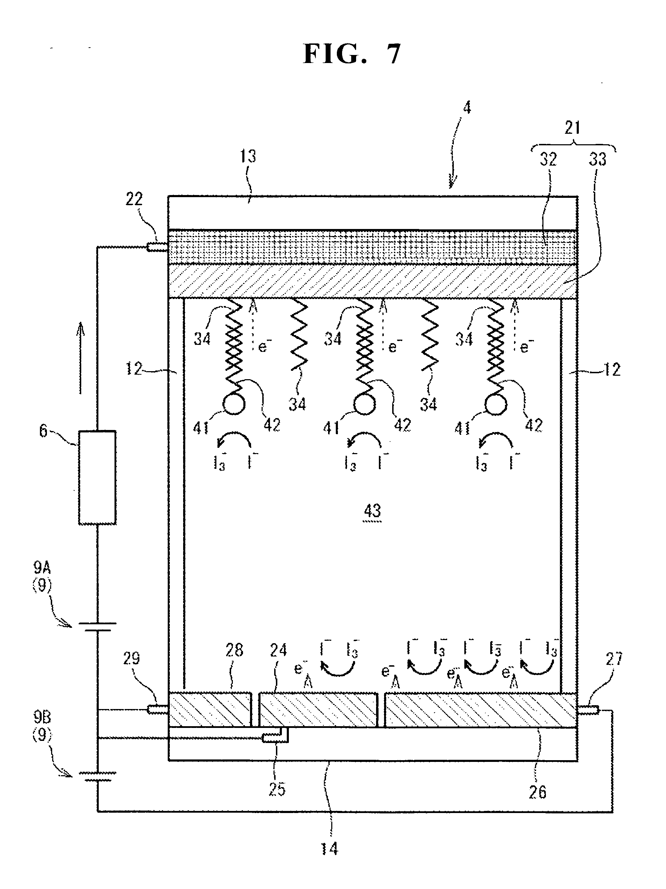

FIG. 7 is a perspective view for schematically showing the test chip 4. The working electrode 21 is composed of both an electroconductive layer 32 formed on the surface of the upper substrate 13 and an electron-receiving layer (a semiconductor layer; a semiconductor electrode) 33 formed on the surface of the electroconductive layer 32. The electrode-receiving layer 33 contains a substance capable of receiving electrons to be emitted by the analyte substance 42-modifying dye 41 upon photoexcitation. The electrode lead 22 of the working electrode 21 is connected to the electroconductive layer 32.

The upper substrate 13 in the embodiment of the present invention is formed of silicon dioxide (SiO₂). The electroconductive layer 32 and the electrode lead 22 each have a double-layered structure consisting of indium tin oxide (ITO) and antimony-doped tin oxide (ATO) and formed on the upper substrate 13 by sputtering. The electron-receiving layer 33 is composed of an oxide semiconductor titanium oxide (TiO₂) and formed on the electroconductive layer 32 by sputtering or the like.

The materials of the upper substrate 13, the electroconductive layer 32, the electrode lead 22 and the electrode-receiving layer 33 are merely illustrate and not limited thereto. For example, the electroconductive layer 32 can be made of metals such as platinum, gold, silver and copper, electroconductive ceramics, metal oxides, and the like. The electron-receiving layer 33 may be made of a substance capable of having energy levels at which it can receive electrons released from the dye 41 upon excitation, and the electron-receiving layer 33 can be made of an element semiconductor such as silicon or germanium, a compound semiconductor such as cadmium sulfide (CdS), an organic semiconductor, or the like.

When the electron-receiving layer 33 itself is composed of an electroconductive material such as ITO or ATO, the electron-receiving layer 33 can also serve as the electroconductive layer 32.

In the embodiment of the present invention, a metal film (a metal layer) (not shown) is formed on the surface of the electron-receiving layer 33 by vapor deposition or the like. The metal film in the embodiment of the present invention is made of gold (Au), but is not particularly limited as long as it is made of a metal capable of binding to a probe 34 described later, and the metal film may be made of platinum, silver, palladium, nickel, mercury or copper. The method of forming a metal film on the surface of the electron-receiving layer 33 may be sputtering, imprinting, screen printing, plating, or a sol-gel process.

The probe 34 for trapping an analyte 42 modified with the dye 41 is immobilized on the electron-receiving layer 33 (metal film). The probe 34 consists of a substance (for example, a nucleic acid, a protein or a peptide) capable of specifically binding to the analyte 42. The probe 34 is immobilized strongly via a covalent bond onto the metal film arranged on the electron-receiving layer 33.

As the dye 41 for modifying the analyte 42, a ruthenium complex is used, and the dye 41 is bound via a peptide bond to the analyte 42. However, the dye 41 is not particularly limited as long as it is a substance to be excited by the light source 5 (see FIG. 2) thereby emitting electrons. For example, the dye 41 may use a metal complex, an organic dye and a quantum dot. Specific examples of the substance that emits electrons upon photoexcitation include metal phthalocyanine , an osmium complex, an iron complex, a zinc complex, a 9-phenylxanthene dye, a cyanine dye, a metallocyanine dye, a xanthene dye, a triphenylmethane dye, an acridine dye, an oxazine dye, a coumarin dye, a merocyanine dye, a rhodacyanine dye, a polymethine dye, a porphyrin dye, a phthalocyanine dye, a rhodamine dye, a xanthene dye, a chlorophyll dye, an eosin dye, a mercurochrome dye, an indigo dye, and a cadmium selenide dye.

The lower substrate 14 is formed of silicon dioxide (SiO₂). The counter electrode 24, the reduction electrode 26 and the reference electrode 28 are formed of platinum (Pt) and arranged on the lower substrate 14 by sputtering. However, the electrodes 24, 26 and 28 may also be composed of metals such as gold, silver, copper, carbon, aluminum, rhodium and indium, oxide semiconductors, electroconductive ceramics, and the like, and their materials are not particularly limited as long as the electrodes are stable to an electrolyte described later. The electrodes may be a multilayer electrode such as an electrode having platinum adhered by sputtering onto an oxide semiconductor or an electrode having gold adhered by vapor-deposition onto TiO₂ (electroconductive ceramic).

### (Detection method of using the detection apparatus)

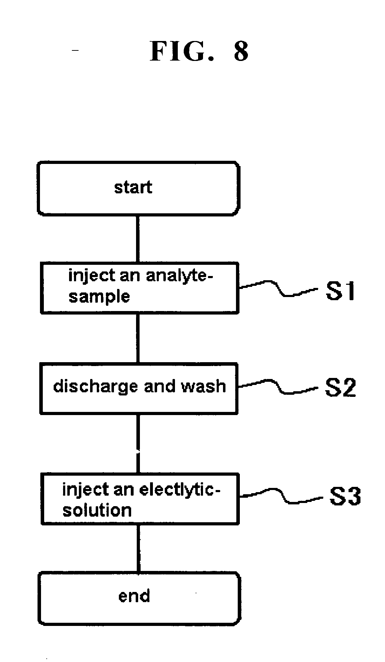

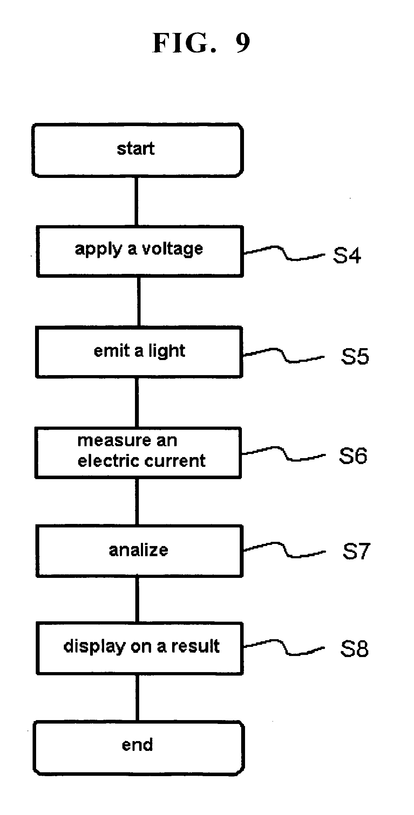

The method of detecting an analyte with the test chip 4 in the detection apparatus 1 will be described. FIG. 8 is a flowchart showing the procedure of injecting an analyte sample into the test chip 4 by the user, and FIG. 9 is a flowchart showing the procedure of detection operation in the detection apparatus 1.

In step S1 in FIG. 8, the user injects an analyte sample via the sample injection port 11 into the test chip 4. The analyte 42 contained in this analyte sample has previously been modified with the dye 41, and as shown in FIG. 7, is trapped by the probe 34 immobilized on the electron-receiving layer 33 of the working electrode 21 (when a nucleic acid is trapped with a nucleic acid probe, this trapping is sometimes referred to as hybridization).

In step S2, the user discharges the solution in the test chip 4 from the sample injection port 11 and then washes the inside of the test chip 4 with a hybridization washing liquid. The probe 34 has been immobilized strongly via a covalent bond onto the metal film arranged on the electron-receiving layer 33, and thus the probe 34 can be prevented from being detached from the electron-receiving layer 33 during the hybridization reaction and during washing the analyte sample.

In step S3, the user injects an electrolyte through the sample injection port 11. This electrolyte may be a mixture containing iodine as an electrolyte, tetrapropylammonium chloride (NPr₄Cl) as a supporting electrolytic salt and an organic solvent consisting of acetonitrile (AN) and ethyl carbonate (EC) mixed in a volume ratio of 6 : 4. When the electrolyte is injected to the test chip 4, the iodine in the electrolyte dissolves the metal film (Au) on the electron-receiving layer 33, and the probe 34 is thereby arranged on the electron-receiving layer 33. Electrons to be released from the dye 41 excited by photoexcitation as described later are received efficiently by the electron-receiving layer 33.

In the embodiment of the present invention, the probe is immobilized by using a metal film, but because it suffices for the probe to be immobilized strongly, a silane coupling agent such as aminopropyltriethoxysilane (APTES) may be used.

Thereafter, the user inserts the test chip 4 into a chip-receiving part 3 of the detection apparatus 1 (see FIG. 1) and instructs the initiation of measurement on the display 2.

In the test chip 4 supplied to the detection apparatus 1, as shown in FIG. 7, the electrode leads 22, 25, 27 and 29 are connected to an ammeter 6 and a power supply 9. The power supply 9 is composed of a first power supply 9A and a second power supply 9B, and the first power supply 9A supplies the working electrode 21 with a potential of 0 V relative to the reference electrode 28, and the second power supply 9B supplies to the reduction electrode 26 with a potential of -0.3 V relative to the reference electrode 28 (step S4 in FIG. 9).

In the embodiment of the present invention, the reference electrode 28 can be omitted. In this case, the first and second power supplies 9A and 9B supply to the working electrode 21 and the reduction electrode 26 with potentials of 0 V and -0.3 V respectively relative to the counter electrode 24. The potentials applied to the working electrode 21 and the reduction electrode 26 can be changed as necessary, and if the oxidation-reduction of the electrolyte described later is sufficiently performed, the application of a potential to the electrodes 21 and 26 is not always necessary.

In step S5 in FIG. 9, the light source 5 applies a light to the dye 41 with which the analyte 42 has been modified, thereby exciting the dye 41. In the embodiment of the present invention, the light source 5 is designed to emit a laser light, and the outer surface of the upper substrate 13 (that is, that side of the upper substrate that is opposite to the electron-receiving layer 33) is irradiated with a laser light. The dye 41 excited by the laser light emits electrons, and the emitted electrons are received by the electron-receiving layer 33. As a result, a current (photocurrent) passes via the electrolyte between the working electrode 21 and the counter electrode 24.

In step S6, the current which due to the movement of electrons from the dye 41 to the electron-receiving layer 33, passes between the working electrode 21 and the counter electrode 24, is measured with the ammeter 6. The current value measured with the ammeter 6 is highly correlated with the number of dyes, and thus the analyte 42 can be quantitatively determined on the basis of the measured current value.

The principle of current flow (electron transfer) between the working electrode 21 and the counter electrode 24 will be described with reference to FIG. 7. When the dye 41 emits electrons upon photoexcitation, iodine (I⁻) in the electrolyte 43 transfer electrons to the dye 41 and turns (I₃⁻) by being oxidized as shown in the following formula:

Formula: 3I⁻ ⇔ I₃⁻ + 2e⁻

(I₃⁻) in the electrolyte 43, generated by oxidation, is transferred through natural diffusion to the counter electrode 24 and receives electrons from the counter electrode 24, thereby being reversibly reduced to (I⁻). In this way, the oxidation-reduction of the electrolyte occurs repeatedly, whereby a current passes between the working electrode 21 and the counter electrode 24.

When the oxidation-reduction reaction described above occurs repeatedly, the concentration of (I⁻) capable of transferring electrons to the dye 41 is decreased, and as a consequence, the flow of electrons from the counter electrode 24 to the working electrode 21 is decreased, so that the sensitivity of detection of the analyte may be deteriorated. In the present invention, therefore, the reduction electrode 26 is arranged separately from the counter electrode 24 in the test chip 4, thereby transferring electrons from the reduction electrode 26 to (I₃⁻) in the electrolyte 43, to reversibly reduce (I₃⁻) to (I⁻). The reduction reaction of (I₃⁻) is thereby promoted, whereby the concentration of (I⁻) can be increased and a current passing between the working electrode 21 and the counter electrode 24 can be increased thereby improving the accuracy of detection of the analyte 42.

In step S7 in FIG. 9, a digital value into which the current value has been converted by the A/D converter 7 is inputted to the controller 8. On the basis of a previously prepared calibration curve showing the relationship between current values and the amounts of the analyte 42, the controller 8 makes a rough estimate of the amount of the analyte 42 in the analyte sample, from the digital value into which the current value has been converted. To indicate on the display 2 the roughly estimated amount of the analyte 42, an image of the detection result is formed.

Then, in step S8, the detection result image formed by the controller 8 is transmitted to, and displayed on the display 2, and the processing is terminated.

In the embodiment described above, the light source 5 is not limited to one emitting a laser light as long as it excites the dye 41, and examples of such light sources include a light-emitting diode (LED), an inorganic electroluminescence element, and an organic electroluminescence element. The light source may be a white light source such as a halogen lamp or a xenon lamp, or an exciting light source having a white light source combined with an optical filter.

The electrodes 21, 24, 26 and 28 may be contacted with one another via an electrolyte and may thus not necessarily be formed on the different substrates 13 and 14, and may be formed on the same substrate. The reduction electrode 26 is not limited to the "comb-shaped" electrode and may be a plate-shaped electrode. However, when the reduction electrode 26 is formed as the "comb-shaped" electrode, the reduction efficiency of the electrolyte can be improved due to structures such as the edge.

### EXAMPLES

### Experiment 1. Verification of the Effect of the Reduction Electrode

The following experiment was conducted to verify the effect of the reduction electrode illustrated above. In this experiment, a value of a photocurrent passing between the working electrode and the counter electrode was measured both when the reduction electrode was used to reduce an electrolyte (Example) and when the reduction electrode was not used (Comparative Example), and the value of a photocurrent was compared between the Example and Comparative Example, to verify the effectiveness of the reduction electrode.

### (Preparation of Electrodes)

Preparation of the electrodes used in this experiment is first described. The working electrodes used in the Examples and Comparative Examples were prepared by forming, by sputtering, a double-layered thin film consisting of indium tin oxide (ITO; thickness 200 nm) and antimony-doped tin oxide (ATO; thickness 100 nm) serving as both an electroconductive layer and an electron-receiving layer, on the surface of a substrate formed of silicon dioxide (SiO₂), and then forming a metal film consisting of gold (thickness 10 nm) on the surface of the electron-receiving layer by vacuum deposition.

Each of the counter electrode, the reduction electrode arranged separetely from the counter electrode and the reference electrode in the Example was prepared by forming a thin film made of platinum (Pt) by sputtering on a substrate made of silicon dioxide (SiO₂). In the Comparative Examples, on the other hand, the counter electrode and the reference electrode only formed on a substrate were used.

### (Immobilization of Probe Substance)

A probe consisting of a DNA (24 bases) having a thiol group was immobilized on the electron-receiving layer (on the metal film) of the working electrode. This operation was performed by dipping the working electrode for a predetermined time (about 14 hours) in an aqueous solution having the DNA (24 bases) dispersed therein, then washing the working electrode with water and drying it. The probe was thereby immobilized on the metal film (Au) via a covalent bond between the thiol group of the DNA probe and the metal film on the electron-receiving layer.

### (Preparation of Analyte)

As the analyte, a dye-bound DNA having a nucleotide sequence complementary to the probe was prepared. As the dye, a ruthenium complex (trade name: Pulsar 650, manufactured by Bio Search Technologies Japan) was used, and the ruthenium complex was bound via a peptide bond to the DNA.

### (Hybridization between the Analyte and the Probe)

By hybridization reaction, the dye-modified analyte was trapped by the probe on the working electrode. This operation was performed by injecting the dye-modified DNA onto the working electrode, heating it at a predetermined temperature (about 45°C) and simultaneously leaving it in a stationary state for a predetermined time (for about 1 hour).

### (Preparation of Electrolyte)

The electrolyte was prepared by dissolving 0.6 M tetrapropylammonium chloride (NPr₄Cl) as a supporting electrolytic salt and 0.06 M iodine as an electrolyte in a solvent of acetonitrile (AN) and ethyl carbonate (EC) mixed in a volume ratio of 6 : 4.

### (Measurement of Photocurrent)

The electron-receiving layer (metal film) of the working electrode was surrounded by a spacing member silicone rubber (thickness 0.2 mm), and 8 µL of the electrolyte was injected into the space formed by the silicone rubber. Then, the working electrode into which the electrolyte had been injected was sealed with the substrate having the counter electrode, the reference electrode and the reduction electrode formed in this order from above. The working electrode, the counter electrode, the reference electrode and the reduction electrode were thereby contacted with the electrolyte.

Voltages of -0.3 V relative to the reference electrode were applied by the power supply to the reduction electrode in the Example. Voltages of 0 V relative to the reference electrode were applied by the power supply to the working electrode in the Example.

In the Comparative Example, on the other hand, a voltage of 0 V relative to the reference electrode was applied to the working electrode. Then, that side of the apparatus that was opposite to the probe-fixed surface of the working electrode was irradiated with a light from a light source. The light source used was one emitting a laser light having a specific wavelength (wavelength about 473 nm, intensity about 13 mW) capable of exciting the dye and was turned on and off at a specific frequency (1 Hz). Then, the photocurrent passing between the working electrode 21 and the counter electrode 24, which was increased or decreased with the laser light to be turned on and off, was measured with an ammeter 6, and simultaneously an increase in the photocurrent was determined.

### (Measurement Result)

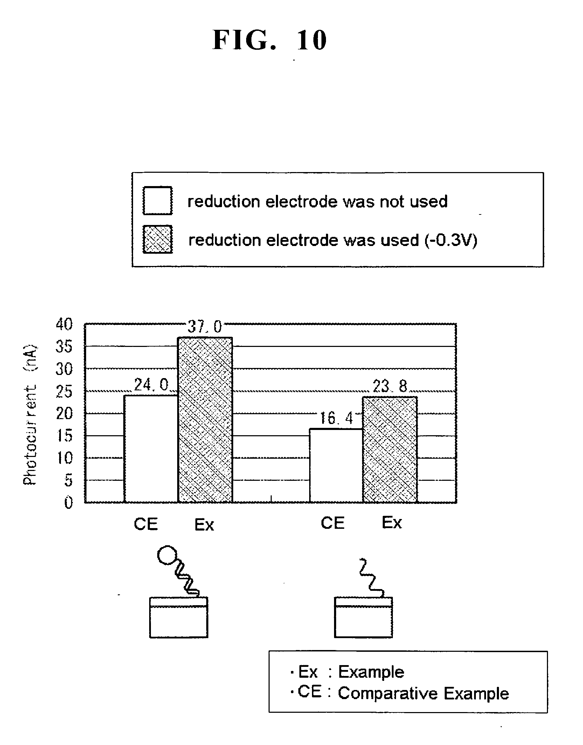

FIG. 10 is a graph showing the measurement result of values of the photocurrent passing between the working electrode and the counter electrode in Experiment 1. In this graph, the shaded bar shows the case where the reduction electrode was used (the Example), and the white bar shows the case where the reduction electrode was not used (the Comparative Example). The measurement results of values of the photocurrent where the step of hybridizing the analyte with the probe was carried out (the left bars in the graph) and not carried out (the right bars in the graph) in the Example and Comparative Example are also shown. When the step of hybridizing the analyte with the probe was not carried out, the electrode itself was excited by irradiation with a light from the light source, and an electrode-derived current passes between the working electrode and the counter electrode and appears as noise.

The photocurrent value where the step of hybridizing the analyte with the probe had been conducted in the Example was 37.0 nA, while the photocurrent value where the step of hybridizing the analyte with the probe had been conducted in the Comparative Example was 24.0 nA. It can thus be seen that when the reduction electrode is arranged as shown in the Example, the value of the photocurrent passing between the working electrode and the counter electrode can be increased and the detection sensitivity of the analyte can be improved.

The photocurrent value where the step of hybridizing the analyte with the probe was not conducted in the Example was 23.8 nA, while the photocurrent value where the hybridizing step was not conducted in the Comparative Example was 16.4 nA. Accordingly, when S/N in the Example is compared with S/N in the Comparative Example, S/N in the Example = 37.0/23.8 = 1.55, while S/N in the Comparative Example = 24.0/16.4 = 1.46. That is, it can be seen that the S/N ratio in the Example can be increased and the detection accuracy of the analyte can be improved.

### Experiment 2. Verification of Change with Time of Photocurrent Value

In this experiment, a change with time in the photocurrent value detected with the working electrode was measured.

### (Preparation of Electrodes)

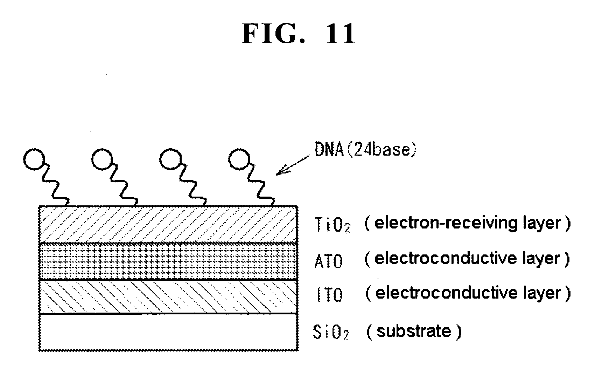

As shown in FIG. 11, the working electrode was prepared by forming, by sputtering, an electroconductive layer consisting of 2 layers, that is, indium tin oxide (ITO; thickness 200 nm) and antimony-doped tin oxide (ATO; thickness 100 nm), on the surface of a substrate formed of silicon dioxide (SiO₂), and then forming an electron-receiving layer consisting of titanium dioxide (TiO₂) on the surface of the electroconductive layer.

The counter electrode, the reference electrode and the reduction electrode were prepared in the same manner as in Experiment 1.

### (Preparation of Analyte)

Without immobilizing a probe, a dye-modified analyte (DNA) was directly immobilized on the surface of the electron-receiving layer 33 in this experiment. For this immobilization, the bonding between a thiol group of the nucleic acid and TiO₂ and the bonding between a phosphate group and TiO₂ were used.

As the dye, the same ruthenium complex (trade name: Pulsar 650, manufactured by Bio Search Technologies Japan) as in Experiment 1 was used, and the ruthenium complex was bound via a peptide bond to a DNA as the analyte.

### (Preparation of Electrolyte)

The electrolyte was prepared in the same manner as in Experiment 1.

### (Measurement of Photocurrent)

As in Experiment 1, potentials of 0 V and -0.3 V relative to the reference electrode were applied to the working electrode and the reduction electrode, respectively. By the same light source as in Experiment 1, the laser light was turned on and off at a frequency of 1 Hz and applied to that side of the working electrode which was opposite to the electron-receiving layer.

### (Measurement Result)

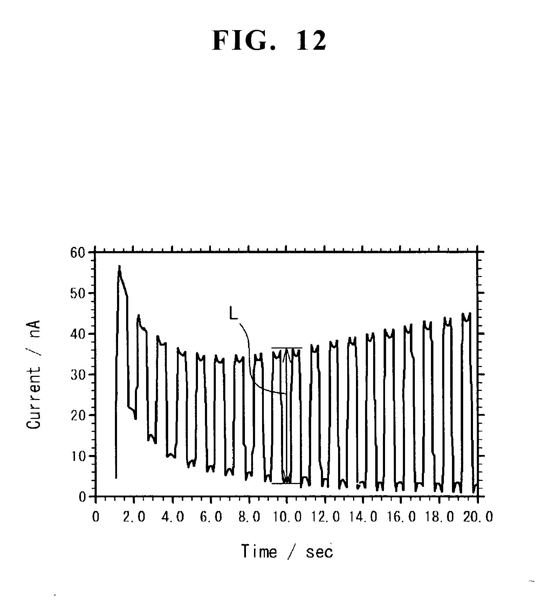

FIG. 12 is a graph showing a change with time in the photocurrent value detected with the working electrode in Experiment 2. The photocurrent detected with the working electrode is increased or decreased with the laser light to be turned on and off. It can be seen that the increase L from "off' to "on" of the light source is increased with time. This is considered due to the fact that the reduced amount of the electrolyte (iodine) in the electric solution is gradually increased due to the presence of the reduction electrode.

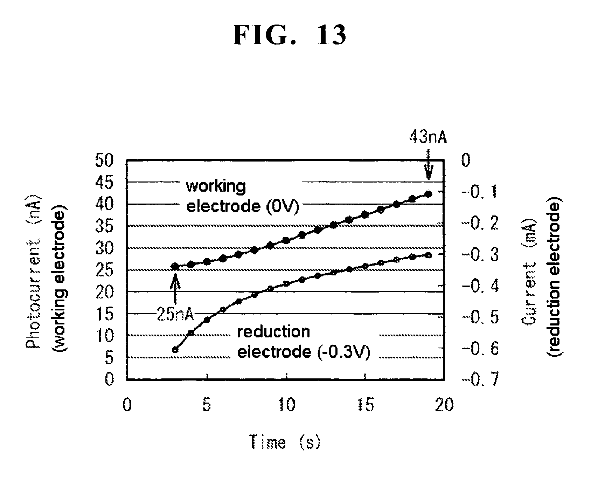

FIG. 13 is a graph showing the photocurrent values detected in the working electrode, and the current values detected in the reduction electrode, in Experiment 2. The respective values show the increase L in the current value in FIG. 12.

From this experiment, it can be seen that just after the measurement is initiated (far left in the graph), the photocurrent value in the working electrode and the current value in the reduction electrode are small and thereafter gradually increased with time. This is considered due to the fact that when a predetermined voltage is applied to the reduction electrode, the amount of the electrolyte to be immediately reduced is small just after the measurement is initiated, and thus the photocurrent value is not immediately increased, and thereafter, the reduction reaction with the reduction electrode becomes gradually activated with time, thus increasing the photocurrent value. Accordingly, it can be seen that after the measurement is initiated, elapse of a certain time is necessary for increasing an increase in the detection sensitivity of the analyte.

It is considered that the photocurrent value just after initiation of the measurement (far left in the graph) hardly undergoes the influence of the reduction electrode and is thus estimated to be almost equal to the photocurrent value in the absence of the reduction electrode. Then, the thus estimated photocurrent is about 25 nA, while the photocurrent value after about 20 seconds is about 43 nA, and it can thus be considered that an about 1.7-fold photocurrent value can be produced by the presence of the reduction electrode.

### Experiment 3. Examination (I) of Potential Applied to the Reduction electrode

In this experiment, a cyclic voltammogram showing the electrochemical behavior of the electrolyte was measured to examine the potential applied by the power supply to the reduction electrode.

In this experiment, the same electrolyte as in Experiments 1 and 2 was used, and an electrode made of platinum (Pt) was used. The electrode was used as a "comb-shaped" electrode (provided with line-shaped portions of 5 µm in width formed with 10-µm pitches) similar to the reduction electrode 26 shown in FIG. 6 and as a "well-shaped" electrode provided with a plurality of wells (wells of 3 µm in well diameter formed with 10-µm pitches).

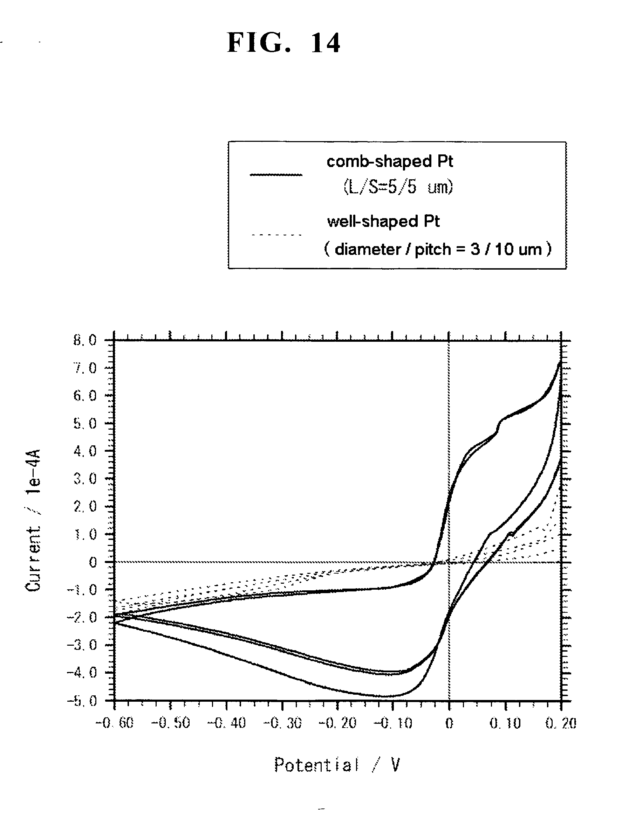

FIG. 14 is a graph showing the measurement result in a cyclic voltammogram of an electrolyte in Experiment 3. A potential from a negative potential (left side in FIG. 14) to a positive potential (right side in FIG. 14) was applied to the electrode; 6 cycles of this operation were carried out in measurement. The sweep rate of the potential was 10 mV/s.

From this result, it can be seen that when the potential applied to the electrode is smaller than approximately 0 V, a minus current, that is, a reduction current has been detected. The potential considered to allow the reduction current to effectively pass through both the "comb-shaped" and "well-shaped" electrodes is in the range of -0.5 V or more and less than 0 V, and it can be estimated that when the potential applied to the reduction electrode is established in this range, the reduction reaction of the electrolyte can be preferably promoted.

### Experiment 4. Examination (II) of Potential Applied to the Reduction Electrode 26

While the potential applied to the reduction electrode was changed in a predetermined range based on the measurement results in Experiment 3, the photocurrent value was measured in this experiment.

### (Preparation of Electrodes)

As the working electrode, an electron-receiving layer made of indium tin oxide (ITO; thickness 200 nm) was formed on a substrate made of silicon dioxide (SiO₂) and then washed by sonication in ethanol (EtOH) for a predetermined time (about 10 minutes). Then, the working electrode was dipped in 1% APTES solution (solvent: toluene), and an excess of APTES was washed away with toluene.

The counter electrode, the working electrode and the reduction electrode were prepared in the same manner as in Experiment 1.

### (Preparation of Analyte)

Four µL of 10 µM dye-modified DNA was dropped onto the working electrode and fixed by dipping for a predetermined time (about 18 hours) in a state sealed with a cover glass at a predetermined temperature (about 37°C). Then, the working electrode was washed with ultrapure water.

### (Measurement of Photocurrent)

First, the same electrolyte (about 8 µL) as in Experiment 3 was injected onto the working electrode.

Further, silica beads of about 10 µm in diameter were arranged in a dispersed state on the working electrode. The silica beads serve as a substitute for the silicone rubber used in Experiment 1 and form a space for injecting the electrolyte between the working electrode and the counter electrodes, the reference electrode or the reduction electrode (also referred to hereinafter as the counter electrode or the like). In Experiment 1, the distance between the working electrode and the counter electrode or the like was set at about 0.2 mm by the silicone rubber, but in this experiment, the distance between the working electrode and the counter electrode or the like was set at about 10 µm that was equal to the diameter of the silica beads.

The working electrode onto which the electrolyte had been injected was sealed with the substrate having the counter electrode, the reference electrode and the reduction electrode formed in this order from above. The working electrode, the counter electrode, the reference electrode and the reduction electrode were thereby contacted with the electrolyte.

By the power supply, a potential of 0 V relative to the reference electrode was applied to the working electrode, and a potential changed with time in the range of 0 V to -0.5 V relative to the reference electrode was applied to the reduction electrode. That side of the working electrode that was opposite to the probe-fixed surface was irradiated with a light from a light source. The light source used was one emitting a laser light having a specific wavelength (wavelength about 488 nm, intensity about 13 mW) capable of exciting the dye.

Then, the photocurrent passing between the working electrode and the counter electrode by light irradiation was measured with an ammeter.

### (Measurement Result)

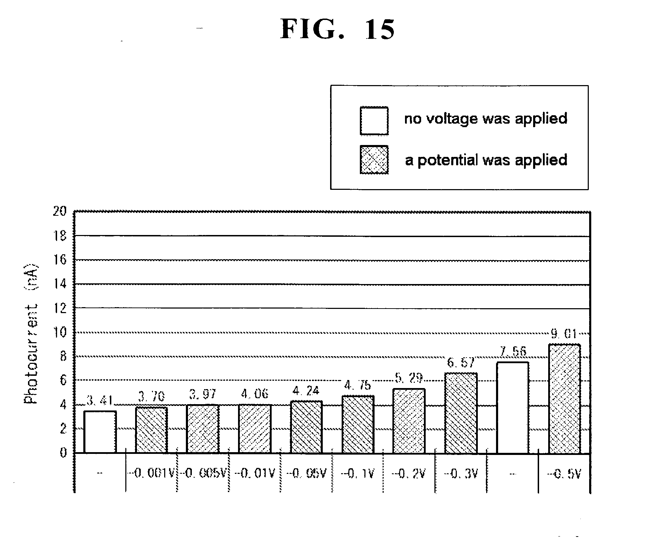

FIG. 15 is a graph showing the measurement result of photocurrent values varying depending on a change in the potential applied to the reduction electrode in Experiment 4. A potential was applied which was changed in the range of 0 V to -0.5 V at predetermined time intervals (about 1 minute) in the order of from the left to right of the graph. However, the far left white bar and the second white bar from the right show photocurrent values where no voltage was applied to the reduction electrode, and other shaded bars show photocurrent values where a potential was applied to the reduction electrode.

As shown in FIG. 15, no voltage was applied to the reduction electrode just after the measurement was initiated, and at this time, the photocurrent value was 3.41 nA. Thereafter, when the potential applied to the reducing voltage was increased toward the negative direction at about 1-minute intervals, it can be seen that the reduction reaction was promoted thereby gradually increasing the photocurrent value. Particularly, when the potential was changed from -0.2 V to -0.3 V, a great increase in photocurrent value of 1 nA or more can be observed. -

After a potential of -0.3 V was applied to the reduction electrode, the application of the potential to the reduction electrode was once stopped, and about 12 minutes thereafter, a potential of -0.5 V was applied again to the reduction electrode. By applying the potential of -0.5 V, the photocurrent can be seen to be further increased. From this experiment, therefore, it can be seen that when the potential applied to the reduction electrode is in the range of less than 0 V and -0.5 V or more, the photocurrent value can be preferably increased and the detection sensitivity of the analyte can be increased.

On the other hand, when the potential applied to the reduction electrode is increased more than -0.5 V toward the negative direction, the electrolyte, the analyte DNA or the DNA probe undergoes electrolytic reaction, which may lead to instable measurement results.

In FIG. 15, the application of a potential was stopped in a step of changing the potential from -0.3 V to -0.5 V, but in this step, the photocurrent value is also increased (the second white bar from the right). This is probably because in the stage until the application of the potential is stopped, the reduction reaction has been promoted with the reduction electrode so that the concentration of (I⁻) in the electrolyte has been increased.

### Experiment 5. Examination of the Space between the Electrodes

In this experiment, the relationship between the photocurrent value and the space between the working electrode and the counter electrode or the like was examined by making the space between the working electrode and the counter electrode or the like different from that in Experiment 1. Specifically, the photocurrent value was measured with a space of about 200 nm formed between the working electrode and the counter electrode, and this measurement value was compared with a photocurrent value measured when a space of an about 200 µm formed therebetween.

### (Manufacture of the working electrode and preparation of the analyte)

Manufacture of the working electrode and preparation of the analyte were carried out in the same manner as in Experiment 4 above.

### (Manufacture of the counter electrode, the reduction electrode, and the reference electrode)

The reduction electrode was prepared in the same manner as for the "well-shaped" electrode (provided with wells of 3 µm in well diameter formed with 10-µm pitches) used in Experiment 3. The counter electrode, the reduction electrode, and the reference electrode were formed in the same manner as in Experiment 1 by forming a thin film made of platinum (Pt) by sputtering on a substrate made of silicon dioxide (SiO₂).

### (Preparation of the electrolyte)

The electrolyte was prepared by dissolving 0.6 M tetrapropylammonium iodine (NPr₄I) as a supporting electrolytic salt and 0.06 M iodine as an electrolyte in a solvent consisting of acetonitrile (AN) and ethyl carbonate (EC) mixed in a volume ratio of 6 : 4.

### (Measurement of photocurrent)

First, the electrolyte (about 1 µL) was dropped onto the working electrode. Then, silica beads of about 200 nm in diameter were arranged in a dispersed state on the working electrode. By the silica beads, a gap (space) for injecting the electrolyte was formed between the working electrode and the counter electrode or the like. In Experiment 1, the space formed by the silicone rubber was about 200 µm (0.2 mm), but in this experiment, the space formed by the silica beads was about 200 nm that was equal to the diameter of the silica beads.

The working electrode onto which the electrolyte had been injected was sealed with the substrate having the counter electrode, the reference electrode and the reduction electrode formed in this order from above. The working electrode, the counter electrode, the reference electrode and the reduction electrode were thereby contacted with the electrolyte.

A potential of 0 V relative to the reference electrode was applied to the working electrode, and a potential of -0.1 V relative to the reference electrode was applied to the reduction electrode. That side of the working electrode which was opposite to the probe-fixed surface was irradiated with a light from a light source. The light source emitted a laser light having a specific wavelength (wavelength about 488 nm, intensity about 13 mW) capable of exciting the dye.

Then, the photocurrent passing between the working electrode and the counter electrode by light irradiation was measured with an ammeter.

### (Measurement Result)

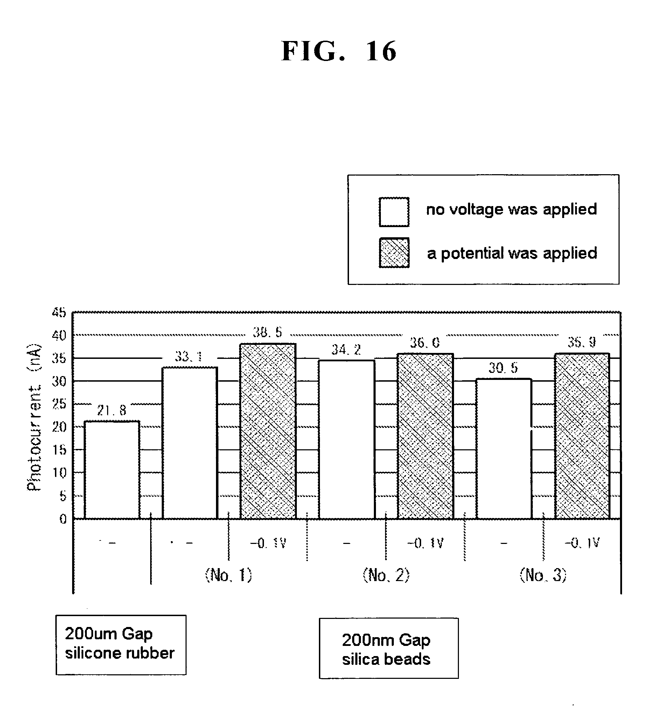

FIG. 16 is a graph showing the measurement result of the photocurrent passing between the working electrode and the counter electrode in Experiment 5. In FIG. 16, the left white bars show the measurement result of the photocurrent where a silicone rubber of about 200 µm in thickness was used and no potential was applied to the reduction electrode. The measurement of the photocurrent where silica beads of about 200 nm in diameter were used was carried out 3 times, and the respective measurement results are shown in Nos. 1 to 3 in FIG. 16. In the respective measurements in Nos. 1 to 3, the photocurrent was measured in 2 patterns, that is, with a potential applied and with no potential applied to the reduction electrode, and the measurement results are shown in the shaded bars and the white bars, respectively.

The measured photocurrent values shown in No. 1 to 3 are compared between the case where a potential was applied to the reduction electrode and the case where no potential was applied to the reduction electrode, the former case shows a higher photocurrent value. Accordingly, it can be judged that the reduction reaction of the electrolyte is promoted by applying a potential to the reduction electrode. When the space between the working electrode and the counter electrode was 200 µm, the measured value was 21.8 nA, while when the space between the working electrode and the counter electrode was 200 nm, the measured value was 33.1 nA (No. 1), and even when no potential is applied to the reduction electrode, the photocurrent value is increased about 1.5-fold. Accordingly, it can be seen that as the space between the working electrode and the counter electrode or the like is decreased, the photocurrent can be measured more effectively. Even if no potential is applied to the reduction electrode, the electrode of a material (platinum in this experiment) capable of maintaining the electrolyte such at a potential as to be reducible in the condition of natural potential can be used as the reduction electrode.

The constitution of the electrode, the electrolyte, the light source or the like used in each experiment described above can be applied to the test chip, the test apparatus and the method in the embodiment of the present invention.

## Claims

1. A method for detecting an analyte modified with a modulator releasing electrons upon photoexcitation, comprising:
trapping an analyte in a sample by using a test chip;
irradiating, with a light for exciting the modulator, the modulator with which the analyte trapped by the probe has been modified, while the working electrode, the counter electrode and the reduction electrode are contacted with an electrolyte medium containing the electrolyte; and
measuring a current which due to movement of electrons from the photoexcited modulator to the working electrode, passes between the working electrode and the counter electrode, while the electrolyte contained in the electrolyte medium is reduced by the reduction electrode;
wherein the test chip comprises:
a working electrode including a semiconductor layer;
a probe being capable of trapping the analyte and being immobilized on the semiconductor layer;
a counter electrode consisting of an electroconductive material; and
a reduction electrode for reducing an electrolyte contained in an electrolyte medium to be contacted with the working electrode and the counter electrode, wherein the reduction electrode is arranged separately from the counter electrode.

2. The method of claim 1, wherein the reduction of the electrolyte is carried out with the reduction electrode under application of a potential.

3. The method of claim 2, wherein the reduction of the electrolyte is carried out with the reduction electrode under application of a potential of -0.5 V or more and less than 0 V relative to the counter electrode.

4. The method of claim 1, wherein the reduction electrode is kept at natural potential capable of reducing the electrolyte.

5. The method of any one of claims 1 to 4, wherein the probe is a nucleic acid, a protein or a peptide.

6. The method of any one of claims 1 to 5, wherein the electrolyte is iodine or an iodide.

7. An apparatus for detecting an analyte modified with a modulator releasing electrons upon photoexcitation, comprising:
a test chip receiving part capable of receiving a test chip;
a light source for irradiating, with a light for exciting the modulator, the modulator with which the analyte has been modified in the test chip received by the test chip receiving part;
a power supply for supplying a potential to the reduction electrode; and
an electric current measuring part for measuring a current which due to movement of electrons from the modulator excited by the light emitted from the light source to the working electrode, passes between the working electrode and the counter electrode;
wherein the testchip comprises:
a working electrode including a semiconductor layer;
a probe being capable of trapping the analyte and being immobilized on the semiconductor layer;
a counter electrode consisting of an electroconductive material; and
a reduction electrode for reducing an electrolyte contained in an electrolyte medium to be contacted with the working electrode and the counter electrode, wherein the reduction electrode is arranged separately from the counter electrode.

8. The apparatus of claim 7, wherein the power supply for supplying a potential applies, to the reduction electrode, a potential of -0.5 V or more and less than 0 V relative to the counter electrode.

9. The apparatus of claim 7 or claim 8, wherein the light source generates a light of wavelength exciting the modulator for modifying an analyte.

10. A test chip for detecting an analyte modified with a modulator releasing electrons upon photoexcitation, comprising:
a working electrode including a semiconductor layer;
a probe being capable of trapping the analyte and being immobilized on the semiconductor layer;
a counter electrode consisting of an electroconductive material; and
a reduction electrode for reducing an electrolyte contained in the electrolyte medium to be contacted with the working electrode and the counter electrode, wherein the reduction electrode is arranged separately from the counter electrode.

11. The test chip of claim 10, wherein the reduction electrode is an electrode which consists of a comb-like structure with several parallel long lines

12. The test chip of claim 10 or claim 11, wherein the electrolyte is iodine or an iodide.

13. The test chip of any one of claims 10 to 12, wherein the probe is a nucleic acid, a protein or a peptide.

14. The test chip of any one of claims 10 to 13, wherein the probe is immobilized by chemical adsorption onto the semiconductor layer.

15. The test chip of claim 14, wherein the probe has a thiol group as a binding group for chemical adsorption to the semiconductor layer.

## Patentansprüche

1. Verfahren zum Detektieren eines Analyten, der mit einem Modulator modifiziert ist, welcher bei Photoanregung Elektronen freisetzt, umfassend:
Festsetzen eines Analyten in einer Probe durch Verwendung eines Testchips;
Bestrahlen des Modulators, mit dem der von der Sonde festgesetzte Analyt modifiziert wurde, mit einem Licht zum Anregen des Modulators, während die Arbeitselektrode, die Gegenelektrode und die Reduktionselektrode mit einem den Elektrolyten enthaltenden Elektrolytmedium in Berührung gebracht werden; und
Messen eines Stroms, der aufgrund einer Bewegung von Elektronen von dem durch Licht angeregten Modulator zu der Arbeitselektrode zwischen der Arbeitselektrode und der Gegenelektrode fließt, während der in dem Elektrolytmedium enthaltene Elektrolyt von der Reduktionselektrode reduziert wird;
wobei der Testchip folgendes umfasst:
eine Arbeitselektrode, die eine Halbleiterschicht umfasst;
eine Sonde, die zum Festsetzen des Analyten in der Lage und auf der Halbleiterschicht immobilisiert ist;
eine Gegenelektrode, die aus elektrisch leitendem Material besteht; und
eine Reduktionselektrode zum Reduzieren eines in einem Elektrolytmedium, das mit der Arbeitselektrode und der Gegenelektrode in Berührung gebracht werden soll, enthaltenen Elektrolyten, wobei die Reduktionselektrode separat von der Gegenelektrode angeordnet ist.

2. Verfahren nach Anspruch 1, wobei die Reduktion des Elektrolyten unter Anlegen eines Potenzials mit der Reduktionselektrode durchgeführt wird.

3. Verfahren nach Anspruch 2, wobei die Reduktion des Elektrolyten durchgeführt wird, während an die Reduktionselektrode ein Potenzial von mindestens -0,5 V und weniger als 0 V relativ zu der Gegenelektrode angelegt ist.

4. Verfahren nach Anspruch 1, wobei die Reduktionselektrode bei einem natürlichen Potenzial gehalten ist, das zum Reduzieren des Elektrolyten in der Lage ist.

5. Verfahren nach einem der Ansprüche 1 bis 4, wobei es sich bei der Sonde um eine Nukleinsäure, ein Protein oder ein Peptid handelt.

6. Verfahren nach einem der Ansprüche 1 bis 5, wobei es sich bei dem Elektrolyten um Iod oder ein Iodid handelt.

7. Apparat zum Detektieren eines Analyten, der mit einem Modulator modifiziert ist, welcher bei Photoanregung Elektronen freisetzt, umfassend:
ein Testchip aufnehmendes Teil, das zum Aufnahmen eines Testchips in der Lage ist;
eine Lichtquelle zum Bestrahlen des Modulators, mit dem der Analyt modifiziert worden ist, in dem Testchip, der von dem einen Testchip aufnehmenden Teil aufgenommen ist, mit einem Licht zum Anregen des Modulators;
eine Stromversorgung zum Abgeben eines Potenzials an die Reduktionselektrode; und
ein einen elektrischen Strom messendes Teil zum Messen eines Stroms, der aufgrund einer Bewegung von Elektronen von dem Modulator, der von dem Licht angeregt ist, welches von der Lichtquelle abgestrahlt wird, zu der Arbeitselektrode zwischen der Arbeitselektrode und der Gegenelektrode fließt;
wobei der Testchip umfasst:
eine Arbeitselektrode, die eine Halbleiterschicht umfasst;
eine Sonde, die zum Festsetzen des Analyten in der Lage und auf der Halbleiterschicht immobilisiert ist;
eine Gegenelektrode, die aus elektrisch leitendem Material besteht; und
eine Reduktionselektrode zum Reduzieren eines in einem Elektrolytmedium, das mit der Arbeitselektrode und der Gegenelektrode in Berührung gebracht werden soll, enthaltenen Elektrolyten, wobei die Reduktionselektrode separat von der Gegenelektrode angeordnet ist.

8. Apparat nach Anspruch 7, wobei die Stromversorgung zum Abgeben eines Potenzials ein Potenzial von mindestens -0,5 V und weniger als 0 V relativ zu der Gegenelektrode an die Reduktionselektrode anlegt.

9. Apparat nach Anspruch 7 oder 8, wobei die Lichtquelle zum Modifizieren eines Analyten ein Licht einer Wellenlänge erzeugt, das den Modulator anregt.

10. Testchip zum Detektieren eines Analyten, der mit einem Modulator modifiziert ist, welcher bei Photoanregung Elektronen freisetzt, umfassend:
eine Arbeitselektrode, die eine Halbleiterschicht umfasst;
eine Sonde, die zum Festsetzen des Analyten in der Lage und auf der Halbleiterschicht immobilisiert ist;
eine Gegenelektrode, die aus elektrisch leitendem Material besteht; und
eine Reduktionselektrode zum Reduzieren eines in einem Elektrolytmedium, das mit der Arbeitselektrode und der Gegenelektrode in Berührung gebracht werden soll, enthaltenen Elektrolyten, wobei die Reduktionselektrode separat von der Gegenelektrode angeordnet ist.

11. Testchip nach Anspruch 10, wobei es sich bei der Reduktionselektrode um eine Elektrode handelt, die aus einer kammartigen Struktur mit mehreren parallelen langen Reihen besteht.

12. Testchip nach Anspruch 10 oder 11, wobei es sich bei dem Elektrolyten um Iod oder ein Iodid handelt.

13. Testchip nach einem der Ansprüche 10 bis 12, wobei es sich bei der Sonde um eine Nukleinsäure, ein Protein oder ein Peptid handelt.

14. Testchip nach einem der Ansprüche 10 bis 13, wobei die Sonde durch chemische Adsorption auf der Halbleiterschicht immobilisiert ist.

15. Testchip nach Anspruch 14, wobei die Sonde eine Thiolgruppe als Bindungsgruppe für die chemische Adsorption an die Halbleiterschicht aufweist.

## Revendications

1. Procédé de détection d'une substance à analyser modifiée avec un modulateur libérant des électrons après photo-excitation, comprenant :
piéger une substance à analyser dans un échantillon en utilisant une puce d'analyse;
irradier, avec une lumière destinée à exciter le modulateur, le modulateur avec lequel la substance à analyser piégée par la sonde a été modifiée, tandis que l'électrode de travail, la contre-électrode et l'électrode de réduction sont mises en contact avec un milieu d'électrolyte contenant l'électrolyte ; et
mesurer un courant qui, en raison du déplacement d'électrons depuis le modulateur photo-excité vers l'électrode de travail, passe entre l'électrode de travail et la contre-électrode, tandis que l'électrolyte contenu dans le milieu d'électrolyte est réduit par l'électrode de réduction ;
la puce d'analyse comprenant :
une électrode de travail incluant une couche de semi-conducteur ;
une sonde capable de piéger la substance à analyser et immobilisée sur la couche de semi-conducteur;
une contre-électrode consistant d'un matériel électro-conducteur ; et
une électrode de réduction permettant de réduire un électrolyte contenu dans un milieu d'électrolyte destinée à être mise en contact avec l'électrode de travail et la contre-électrode, l'électrode de réduction étant disposée séparément de la contre-électrode.

2. Procédé selon la revendication 1, la réduction de l'électrolyte étant effectuée avec l'électrode de réduction sous application d'un potentiel.

3. Procédé selon la revendication 2, la réduction de l'électrolyte étant effectuée avec l'électrode de réduction sous application d'un potentiel de -0,5 V ou plus et inférieur à 0 V par rapport à la contre-électrode.

4. Procédé selon la revendication 1, l'électrode de réduction étant conservée à un potentiel naturel capable de réduire l'électrolyte.

5. Procédé selon l'une quelconque des revendications 1 à 4, la sonde étant un acide nucléique, une protéine ou un peptide.

6. Procédé selon l'une quelconque des revendications 1 à 5, l'électrolyte étant de l'iode ou un iodure.

7. Appareil de détection d'une substance à analyser modifiée avec un modulateur libérant des électrons après photo-excitation, comprenant :
une partie de réception de puce d'analyse capable de recevoir une puce ;
une source lumineuse pour irradier, avec une lumière permettant d'exciter le modulateur, le modulateur avec lequel la substance à analyser a été modifiée dans la puce reçue par la partie de réception de puce d'analyse;
une source d' alimentation pour fournir un potentiel à l'électrode de réduction ; et
une partie de mesure de courant électrique pour mesurer un courant qui, en raison du déplacement d'électrons depuis le modulateur excité par la lumière émise par la source lumineuse vers l'électrode de travail, passe entre l'électrode de travail et la contre-électrode ;
la puce d'analyse comprenant :
une électrode de travail incluant une couche de semi-conducteur ;
une sonde capable de piéger la substance à analyser et immobilisée sur la couche de semi-conducteur ;
une contre-électrode consistant d'un matériel électro-conducteur ; et
une électrode de réduction pour réduire un électrolyte contenu dans un milieu d'électrolyte destinée à être mise en contact avec l'électrode de travail et la contre-électrode, l'électrode de réduction étant disposée séparément de la contre-électrode.

8. Appareil selon la revendication 7, la source d'alimentation électrique destinée à l'application d'un potentiel appliquant, à l'électrode de réduction, un potentiel de -0,5 V ou plus et inférieur à 0 V par rapport à la contre-électrode.

9. Appareil selon la revendication 7 ou 8, la source lumineuse produisant une lumière dont la longueur d'onde excite le modulateur pour modifier une substance à analyser.

10. Puce d'analyse pour la détection d'une substance à analyser modifiée avec un modulateur libérant des électrons après photo-excitation, comprenant :
une électrode de travail incluant une couche de semi-conducteur ;
une sonde capable de piéger la substance à analyser et immobilisée sur la couche de semi-conducteur ;
une contre- électrode consistant d'un matériel électro-conducteur ; et
une électrode de réduction pour réduire un électrolyte contenu dans un milieu d'électrolyte destinée à être mise en contact avec l'électrode de travail et la contre-électrode, l'électrode de réduction étant disposée séparément de la contre-électrode.

11. Puce d'analyse selon la revendication 10, l'électrode de réduction étant une électrode qui consiste en une structure en peigne avec plusieurs longues lignes parallèles.

12. Puce d'analyse selon la revendication 10 ou 11, l'électrolyte étant de l'iode ou un iodure.

13. Puce d'analyse selon l'une quelconque des revendications 10 à 12, la sonde étant un acide nucléique, une protéine ou un peptide.

14. Puce d'analyse selon l'une quelconque des revendications 10 à 13, la sonde étant immobilisée par adsorption chimique sur la couche de semi-conducteur.

15. Puce d'analyse selon la revendication 14, la sonde ayant un groupe thiol comme groupe de liaison pour l'adsorption chimique sur la couche de semi-conducteur.
